# EUROPEAN PATENT SPECIFICATION

(11) **EP 0 910 414 B1**
(45) Date of publication and mention of the grant of the patent: **02.01.2003**
(21) Application number: 97924128.8
(22) Date of filing: 29.05.1997
(51) Int. Cl.: A61K 49/00

(54) **A DIAGNOSTIC TEST FOR SCHIZOPHRENIA, USING NIACIN**
TEST ZUR DIAGNOSE VON SCHIZOPHRENIE MITTELS NIACIN
TEST DIAGNOSTIQUE UTILE DANS LA SCHIZOPHRENIE A L'AIDE DE L'ACIDE NICOTINIQUE

(30) Priority: 29.05.1996 GB 9610971
(43) Date of publication of application: 28.04.1999
(73) Proprietor: Scarista Limited, Isle of Man (GB)
(72) Inventor: GLEN, Alexander, Iain, Munro, Kincraig PH21 1NQ (GB); WARD, Pauline, Elizabeth, Inverness IV2 4AY (GB); HORROBIN, David, Frederick, Scotia Phar. Ltd.,, Stirling FK9 4TZ (GB)
(74) Representative: Wakerley, Helen Rachael
(86) International application number: GB9701461
(87) International publication number: WO97045145

(56) References cited:
- KONINKLIJKE NEDERLANDSE MAATSCHAPPIJ TER BEVORDERING DER PHARMACIE: "INFORMATORIUM MEDICAMENTORUM, part IIA" 1985 , KONINKLIJKE NEDERLANDSE MAATSCHAPPIJ TER BEVORDERING DER PHARMACIE , DEN HAAG, NL XP002047025 see page 1033 especially paragraph Menthoneurin liniment
- CHAN SY ET AL: "Quantitative evaluation of drug-induced erythema by using a tristimulus colour analyzer: experimental design and data analysis." SKIN PHARMACOL, 1993, VOL. 6, NO. 4, PAGE(S) 298-312, CH, XP002047014
- ALBERY WJ ET AL: "Percutaneous absorption: in vivo experiments." J PHARM PHARMACOL, MAR 1979, VOL. 31, NO. 3, PAGE(S) 140-7, GB, XP002047015
- DOWD PM ET AL: "Hexyl-nicotinate-induced vasodilation in normal human skin." DERMATOLOGICA, 1987, VOL. 174, NO. 5, PAGE(S) 239-43, CH, XP002047016
- EMDEN J ET AL: "Vergleich physikalischer Parameter von Hautdurchblutungsanderungen nach epicutaner Applikation von Nicotinsaurebenzylester. Calorische Messungen, Untersuchungen der Erythembildung, der Perspiration und des Hautwiderstandes." ARCH DERMATOL FORSCH, 1971, VOL. 241, NO. 4, PAGE(S) 353-63, DE, XP002047017
- REALDON N ET AL: "Influence of ointment formulation on skin erythema induced by nicotinate esters." PHARMAZIE, SEP 1995, VOL. 50, NO. 9, PAGE(S) 603-6, DE, XP002047018
- GEAN CJ ET AL: "Cutaneous responses to topical methyl nicotinate in black, oriental, and caucasian subjects." ARCH DERMATOL RES, 1989, VOL. 281, NO. 2, PAGE(S) 95-8, DE, XP002047019
- LIN A ET AL: "The niacin challenge test in schizophrenia: past, present and future." PROSTAGLANDINS LEUKOT ESSENT FATTY ACIDS, AUG 1996, VOL. 55, NO. 1-2, PAGE(S) 17-9, SCOTLAND, XP002047020
- MICHAELSSON G ET AL: "Abnormal cutaneous reactions in dermatitis herpetiformis." ACTA DERM VENEREOL, 1975, VOL. 55, NO. 5, PAGE(S) 351-8, SE, XP002047021
- WILSON DW ET AL: "Niacin skin flush is not diagnostic of schizophrenia." BIOL PSYCHIATRY, AUG 1986, VOL. 21, NO. 10, PAGE(S) 974-7, US, XP002047022
- FIEDLER P ET AL: "Niacin -induced flush as a measure of prostaglandin activity in alcoholics and schizophrenics." BIOL PSYCHIATRY, NOV 1986, VOL. 21, NO. 13, PAGE(S) 1347-50, US, XP002047023
- RYBAKOWSKI J ET AL: "Niacin test in schizophrenia and affective illness." BIOL PSYCHIATRY, APR 15 1991, VOL. 29, NO. 8, PAGE(S) 834-6, US, XP002047024

## Description

### Field of Invention

The invention relates to a diagnostic test for use in schizophrenia.

### Background

Schizophrenia is a common psychiatric disease which in most populations affects about 1% of the population. There are many different clinical presentations of the disease which can be broadly divided into two main groups, the "positive" features and the "negative" or "deficit" features. The positive features include auditory hallucinations, bizarre behaviour, thought disorder and sometimes paranoia. The negative features include social and emotional withdrawal and an absence of emotional responses to what is happening in the individual's environment. Some of these features are shared by other psychiatric disorders and, especially in the early years of the disease, it may be difficult to distinguish schizophrenia from depression, manic-depression, drug-induced psychoses and psychoses related to alcoholism. Only the long term course allows a diagnosis of schizophrenia to be made with reasonable certainty. This diagnostic uncertainty in the early stages of the disease has important practical consequences because therapies for the different disorders differ and the uncertainty may delay, sometimes for many years, the introduction of optimum therapy. A biochemically based test which would improve early diagnosis would therefore be of great value.

There is evidence that in schizophrenia there is a biochemical abnormality in the metabolism of the fatty acids of membrane phospholipids to prostaglandins (Horrobin DF, Lancet 1:936-7, 1977; Horrobin DF, Glen AIM, Vaddadi KS, Schizophrenia Research, 1994). In particular, in schizophrenics with the deficit syndrome there is a substantial reduction in the concentrations of arachidonic acid (AA) and docosahexaenoic acid (DHA) in red cell membranes (European Patent Application 0599576). There are also peculiarities in the cPLA2 (phospholipase A2) gene structure (PCT patent application No.97/04127).

Niacin (nicotinic acid) is a vitamin which, when given in a large oral dose, causes marked flushing of the skin over the head, upper body and arms. This flushing is due to vasodilation caused by the release of prostaglandin D₂ from AA. In 1980, Horrobin noted that whereas most normal individuals flush markedly in response to 100-300mg niacin given orally, a substantial proportion of schizophrenics fail to flush. Many but not all of the non-flushing individuals were patients with the deficit syndrome. Horrobin therefore suggest that a flushing response to oral niacin might be used as to assist diagnosis of schizophrenia (Horrobin DF, Journal of Orthomolecular Psychiatry, 9: 33-34, 1980). The idea was that individuals who showed a pattern of behaviour possibly indicative of schizophrenia and who failed to flush in response to niacin would almost certainly have schizophrenia. Individuals who did flush might or might not have the disease. However, in non-flushers appropriate therapy could be introduced without delay. Horrobin also noted that some patients whose schizophrenia improved on being treated with essential fatty acids shifted from a non-flushing to a flushing response and he suggested that flushing could be used as an indicator of improvement in response to treatment. Other investigators have confirmed that a substantial proportion of schizophrenics fail to respond to a fixed oral dose of niacin by flushing (Rybakpwski J et al, Biological Psychiatry, 29: 834-61, 1990, Hudson J et al, Biological Psychiatry, 41: 507-13, 1997). However, there are several drawbacks to the oral test. The flushing reaction is usually accompanied by pricking and tingling sensations which can be quite distressing and there can be a fall in blood pressure. In particular, in paranoid patients the responses may arouse suspicion and hostility. Further, the oral treatment involves a single, all or nothing response which is difficult if not impossible to quantify.

### The Invention

We have now surprisingly found that the oral test can be replaced by a simple test involving the application of different concentrations of nicotinic acid or its C₁-C₁₀ alkyl esters in any effective form, or even a single chosen concentration, to the skin. This is easy to apply, and causes no distress. The test is quickly read, for example in 3-6 minutes with a negative reaction desirably checked after 15 minutes in contrast to the 30-60 minutes which may be required for the oral test. Further, it is not affected by whether or not the individual being tested has a full stomach, and it can be made quantitative.

The invention in its various aspects is set out fully in the claims, to which reference should be made, but may be summarised as use of nicotinic acid or its C1-C10 alkyl esters in the form of topical preparations, in the manufacture of medicaments for monitoring and diagnosis of schizophrenia and devices comprising said topical preparations.

The test involves the topical application of niacin solutions, preferably in a carrier such as gauze, cotton, wax or appropriate fibre or absorbent material, to the skin of any part of the body, but conveniently the forearm or upper arm. A range of solutions may be used from 0.00001M to 1 molar. A single concentration may be chosen and used but desirably use is made of a range of steps varying from two to twenty. We have found it convenient to use a range of four solutions of 0.1, 0.01, 0.001 and 0.0001M, e.g. 0.05 ml of each, but other concentrations within the range and other numbers of steps can be used as found appropriate, both in diagnosis and in monitoring as discussed above. It is particularly convenient to incorporate the niacin-impregnated patches into a plastic or other strip, wherein the individual patches range for example from 1 to 20mm in diameter, and wherein the patches are in individual wells or depressions which may be anything from 0.5mm to 20mm apart, preferably 2-10mm apart. The wells or depressions may be sealed by another plastic or other strip which isolates them from the atmosphere and which can be removed immediately prior to testing. The strip with the open wells can then be applied to the skin surface with light pressure and removed after an appropriate time which may be e.g. 0.5 minutes up to 10 or 15 minutes but preferably 3 to 6 minutes.

In normal individuals, after a short time, an adequate topical concentration of niacin produces both redness (flushing) of the skin and skin swelling (oedema). Both of these effects are due to vasodilatation of the small blood vessels of the skin. The redness may be scored in relation to the adjacent unaffected skin on an appropriate scale which can be either "present" or "absent", or can be semi-quantitative, such as mild, moderate or marked, or can be made fully quantitative by use of a spectrophotometer or other appropriate instrument e.g. a blood flow meter. The oedema can likewise be scored as present or absent or can be scored semi-quantitatively or quantitatively.

We have applied this test to 38 patients with schizophrenia and 22 controls, using niacin concentrations of 0.1, 0.01, 0.001 and 0.0001M, 0.05 ml of each, and time intervals of 0, 5, 10,15, 20 and 25 minutes after application of the niacin containing patches. We have also used a scoring system in which the degree of redness under each patch was scored as 0 = no change, 1 = mild redness, 2 = moderate redness and 3 = marked redness. When the test is used commercially, sample colour charts may show how the test can be used with individuals of varying skin colour. With particularly dark-skinned individuals, oedema or a measurement of skin surface temperature or heat loss may be required to give the best results.

Table 1 shows the scores obtained in the two groups at 5 minutes after application of the test, a time showing good discrimination between the groups:

**Table 1**

| Percentages of normal controls (n=22) and of schizophrenics (n=38) responding at 5 minutes to the application of topical niacin with absent [0], mild [1], moderate [2] or marked [3] reddening of the skin, C=controls and S=schizophrenics. | | | | | | | | |
|---|---|---|---|---|---|---|---|---|
| Niacin Concentrations | | | | | | | | |
| | 0.1M | | 0.01M | | 0.001M | | 0.000.M | |
| | C | S | C | S | C | S | C | S |
| Response | | | | | | | | |
| Absent [0] | 4.5 | 13.5 | 9.1 | 41.2 | 31.8 | 67.6 | 35.3 | 88.2 |
| Mild [1] | 9.1 | 37.8 | 13.6 | 41.2 | 9.1 | 29.4 | 29.4 | 8.8 |
| Moderate [2] | 27.3 | 43.2 | 45.5 | 17.6 | 40.9 | 2.9 | 17.6 | 2.9 |
| Marked [3] | 59.1 | 5.4 | 31.8 | 0 | 18.2 | 0 | 17.6 | 0 |

As may be seen, the three lower concentrations do not give a marked reaction in any of the schizophrenics. Even a moderate reaction, at the two lower concentrations, is shown by very few of the schizophrenics, the great majority showing a mild reaction or none. In contrast, only 4.5% of the normal individuals failed to give a response even to the highest concentration. Given the difficulty of making a secure diagnosis of schizophrenia, the indication given by the new test is thus highly valuable, for use in conjunction with other criteria. A valuable feature is that the test, as with oral niacin (Rybakowski J et al, Biological Psychiatry, loc. cit.) may be expected to give a response in depressives the same as for normal individuals.

Suitably the niacin is in aqueous solution in the form of a C₁ to C₁₀ alkyl ester such as the methyl or hexyl ester but any effective form that is to say a form passing the skin and eliciting the flushing reaction in normal individuals, may be used. Nicotinamide (niacinamide), which does not elicit the reaction, is not suitable.
In the drawings:-
Figure 1 is a simple device for application by hand;
Figure 2 is a cuff-style device with a hook and loop ("Velcro" trade mark) fastening; and
Figure 3 is a section of part of Figure 1, taken axially of the strip at the right hand end as seen.

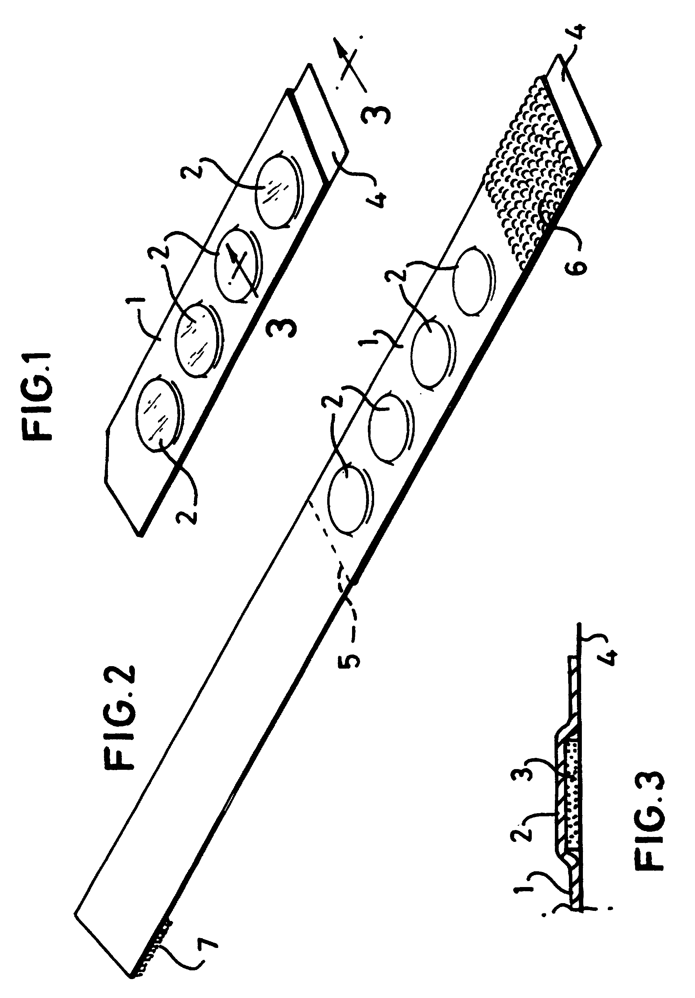

In the drawings, which are merely of examples of ways of using the invention, a moulded flexible plastics strip 1 has wells 2, each holding a gauze pad 3 impregnated with methyl nicotinate solution at the successive concentrations of Table 1. The pads are 16 mm diameter and approximately 1.5 mm thick. A backing strip 4 is secured by pressure sensitive adhesive, to be peeled off when the device is to be used. After peeling, the device is applied to the body, conveniently to the forearm, with the pads in contact with the skin. The device may be held under finger pressure (Figure 1, one finger to a pad) or by the fastening (Figure 2), the two parts of which are indicated at 6 and 7, and the extent of the backing strip at 5. After for example 5 minutes, the device is removed and the skin reaction assessed as already described..

## Claims

1. Use of nicotinic acid or its C₁ or C₁₀ alkyl esters in any effective form as a topical preparation giving absorption of the niacin in any effective form through the skin, with production of a differential effect as between schizophrenics and non-schizophrenics in redness and swelling of the skin, in the preparation of a composition for use in a method of diagnosing or monitoring schizophrenia.

2. Use according to claim 1 in which the differential effect is observable visually in terms of colour or by temperature measurement or heat loss, to be scored against a pre-prepared scale.

3. Use according to claim 1 in which the niacine ester is a C₁₋₁₀ alkyl ester.

4. Use according to claim 3 in which the niacin ester is a C₁₋₆ alkyl ester.

5. Use according to claim 4 in which the niacin ester is the methyl ester.

6. Use according to any preceding claim wherein the niacin or ester is present in the preparation in a concentration of 0.00001 M to 1.0 M.

7. Use according to claim 6 in which the niacin or ester is present in a concentration of 0.0001 M to 0.1 M.

8. Use of two or more topical preparations containing niacin, each preparation containing niacin in a different concentration, with production of a differential effect as between schizophrenics and non-schizophrenics in redness and swelling of the skin, in the preparation of a composition for use in a method of diagnosing or monitoring schizophrenia.

9. Use of two or more topical preparations containing a C₁₋₁₀ alkyl ester of niacin, each preparation containing niacin ester in a different concentration, with production of a differential effect as between schizophrenics and non-schizophrenics in redness and swelling of the skin, in the preparation of a composition for use in a method of diagnosing or monitoring schizophrenia.

10. A device for the diagnosis or monitoring of schizophrenia comprising a plurality of topical preparations, each containing niacin in a concentration selected from the group consisting of: 0.1M; 0.01M; 0.001M and 0.0001M, each topical preparation containing the niacin in any effective form giving absorption of the niacin through the skin.

11. A device for the diagnosis or monitoring of schizophrenia comprising a plurality of topical preparations containing a C₁₋₁₀ alkyl ester of niacin in concentrations selected from the group consisting of: 0.1M; 0.01M; 0.001M and 0.0001M, each topical preparation containing niacin ester in any effective form giving absorption of niacin ester through the skin.

12. A device according to claim 10 or 11 further comprising a flexible strip having a plurality of wells, in which each of the topical preparations is contained in one of the wells, each well contains a different concentration of niacin or ester, and the flexible strip is adapted to application to the skin of a patient and to bringing each of the topical preparations into contact with the skin.

13. A device according to claim 12 in which the flexible strip is sealed with a removable cover film.

14. A device for the diagnosis or monitoring of schizophrenia comprising: a topical preparation containing niacin in any effective form in a concentration in the range from 0.00001M to 1M giving absorption of the niacin through the skin; and a quantifying aid for scoring skin flushing reaction.

15. A device for the diagnosis or monitoring of schizophrenia comprising: a topical preparation containing a C₁₋₁₀ alkyl ester of niacin in any effective form in a concentration in the range from 0.00001M to 1M giving absorption of niacin ester through the skin; and a quantifying aid for scoring skin flushing reaction.

16. A device according to claim 14 or 15 in which the quantifying aid is a colour chart.

## Patentansprüche

1. Verwendung von Nicotinsäure oder ihren C₁ oder C₁₀ Alkylestern in einer beliebigen wirksamen Form als topisches Präparat, das eine Absorption des Niacins in einer beliebigen wirksamen Form durch die Haut erbringt, wobei bei Schizophrenen und Nicht-Schizophrenen eine unterschiedliche Wirkung hinsichtlich einer Rötung und Schwellung der Haut erzeugt wird, zur Herstellung einer Zusammensetzung zur Verwendung in einem Verfahren zur Diagnose oder Überwachung von Schizophrenie.

2. Verwendung nach Anspruch 1, wobei die unterschiedliche Wirkung visuell anhand der Farbe oder durch eine Temperaturmessung oder einen Wärmeverlust beobachtet werden kann, was gegenüber einer zuvor aufgestellten Skala zu bewerten ist.

3. Verwendung nach Anspruch 1, wobei der Niacinester ein C₁₋₁₀ Alkylester ist.

4. Verwendung nach Anspruch 3, wobei der Niacinester ein C₁₋₆ Alkylester ist.

5. Verwendung nach Anspruch 4, wobei der Niacinester Methylester ist.

6. Verwendung nach einem der vorherigen Ansprüche, wobei das Niacin oder der Ester in einer Konzentration zwischen 0,00001 M und 1,0 M in dem Präparat vorliegt.

7. Verwendung nach Anspruch 6, wobei das Niacin oder der Ester in einer Konzentration zwischen 0,0001 M und 0,1 M vorliegt.

8. Verwendung von zwei oder mehreren topischen niacinhaltigen Präparaten, wobei jedes Präparat Niacin in einer anderen Konzentration enthält, wobei bei Schizophrenen und Nicht-Schizophrenen eine unterschiedliche Wirkung hinsichtlich einer Rötung und Schwellung der Haut erzeugt wird, zur Herstellung einer Zusammensetzung zur Verwendung in einem Verfahren zur Diagnose und Überwachung von Schizophrenie.

9. Verwendung von zwei oder mehreren topischen Präparaten, die einen C₁₋₁₀ Alkylester von Niacin enthalten, wobei jedes Präparat einen Niacinester in einer anderen Konzentration enthält, wobei bei Schizophrenen und Nicht-Schizophrenen eine unterschiedliche Wirkung hinsichtlich einer Rötung und Schwellung der Haut erzeugt wird, zur Herstellung einer Zusammensetzung zur Verwendung in einem Verfahren zur Diagnose und Überwachung von Schizophrenie.

10. Vorrichtung zur Diagnose oder Überwachung von Schizophrenie, umfassend eine Mehrzahl topischer Präparate, die Niacin jeweils in einer Konzentration enthalten, die ausgewählt wird aus der Gruppe bestehend aus: 0,1 M; 0,01 M; 0,001 M und 0,0001 M, wobei jedes topische Präparat das Niacin in einer beliebigen wirksamen Form enthält, die eine Absorption des Niacins durch die Haut erbringt.

11. Vorrichtung zur Diagnose oder Überwachung von Schizophrenie, umfassend eine Mehrzahl topischer Präparate, die einen C₁₋₁₀ Alkylester von Niacin in einer Konzentration enthalten, die ausgewählt wird aus der Gruppe bestehend aus: 0,1 M; 0,01 M; 0,001 M und 0,0001 M, wobei jedes topische Präparat den Niacinester in einer beliebigen wirksamen Form enthält, die eine Absorption des Niacinesters durch die Haut erbringt.

12. Vorrichtung nach Anspruch 10 oder 11, ferner umfassend einen flexiblen Streifen mit einer Mehrzahl von Mulden, wobei die jeweiligen topischen Präparate in einer der Mulden enthalten sind, jede Mulde eine unterschiedliche Konzentration des Niacins oder Esters enthält und der flexible Streifen dafür vorgesehen ist, auf die Haut eines Patienten aufgebracht zu werden und die jeweiligen topischen Präparate mit der Haut in Kontakt zu bringen.

13. Vorrichtung nach Anspruch 12, wobei der flexible Streifen mit einer entfernbaren Abdeckfolie versiegelt ist.

14. Vorrichtung zur Diagnose oder Überwachung von Schizophrenie, umfassend: ein topisches Präparat, das Niacin in einer beliebigen wirksamen Form in einer Konzentration zwischen 0,00001 M und 1 M enthält und eine Absorption des Niacins durch die Haut erbringt; und eine Quantifizierungshilfe zur Bewertung der Hautrötungsreaktion.

15. Vorrichtung zur Diagnose oder Überwachung von Schizophrenie, umfassend: ein topisches Präparat, das einen C₁₋₁₀-Alkylester von Niacin in einer beliebigen wirksamen Form in einer Konzentration zwischen 0,00001 M und 1 M enthält und eine Absorption des Niacinesters durch die Haut erbringt; und eine Quantifizierungshilfe zur Bewertung der Hautrötungsreaktion.

16. Vorrichtung nach Anspruch 14 oder 15, wobei die Quantifizierungshilfe eine Farbtabelle ist.

## Revendications

1. Utilisation d'acide nicotinique ou de ses esters alkyliques C₁ ou C₁₀ sous une forme efficace quelconque en tant que préparation à usage local procurant l'absorption de la niacine sous une forme efficace quelconque à travers la peau, avec la production d'un effet différentiel en rougeur et tuméfaction de la peau entre schizophrènes et non schizophrènes, dans la préparation d'une composition à utiliser dans une méthode pour diagnostiquer ou contrôler la schizophrénie.

2. Utilisation selon la revendication 1, dans laquelle l'effet différentiel est observable visuellement en terme de couleur ou par mesure de température ou perte de chaleur, à noter par rapport à une échelle préparée d'avance.

3. Utilisation selon la revendication 1, dans laquelle l'ester de niacine est un ester alkylique C₁-C₁₀.

4. Utilisation selon la revendication 3, dans laquelle l'ester de niacine est un ester alkylique C₁-C₆.

5. Utilisation selon la revendication 4, dans laquelle l'ester de niacine est l'ester méthylique.

6. Utilisation selon l'une quelconque des revendications précédentes, dans laquelle la niacine ou l'ester est présent dans la préparation en une concentration de 0,00001 M à 1,0 M.

7. Utilisation selon la revendication 6, dans laquelle la niacine ou l'ester est présent en une concentration de 0,0001 M à 0,1 M.

8. Utilisation de deux ou plusieurs préparations à usage local contenant de la niacine, chaque préparation contenant de la niacine en une concentration différente, avec la production d'un effet différentiel en rougeur et tuméfaction de la peau entre schizophrènes et non schizophrènes, dans la préparation d'une composition à utiliser dans une méthode pour diagnostiquer ou contrôler la schizophrénie.

9. Utilisation de deux ou plusieurs préparations à usage local contenant un ester alkylique C₁-C₁₀ de niacine, chaque préparation contenant de l'ester de niacine en une concentration différente, avec la production d'un effet différentiel en rougeur et tuméfaction de la peau entre schizophrènes et non schizophrènes, dans la préparation d'une composition à utiliser dans une méthode pour diagnostiquer ou contrôler la schizophrénie.

10. Dispositif pour diagnostiquer ou contrôler la schizophrénie, comprenant une pluralité de préparations à usage local, contenant chacune de la niacine en une concentration sélectionnée parmi le groupe consistant en : 0,1 M; 0,01 M; 0,001 M et 0,0001 M, chaque préparation à usage local contenant la niacine sous une forme efficace quelconque procurant l'absorption de la niacine à travers la peau.

11. Dispositif pour diagnostiquer ou contrôler la schizophrénie, comprenant une pluralité de préparations à usage local contenant un ester alkylique C₁-C₁₀ de niacine en des concentrations sélectionnées parmi le groupe consistant en : 0,1 M; 0,01 M; 0,001 M et 0,0001 M, chaque préparation à usage local contenant de l'ester de niacine sous une forme efficace quelconque procurant l'absorption d'ester de niacine à travers la peau.

12. Dispositif selon la revendication 10 ou 11, comprenant en outre une bande flexible ayant une pluralité de puits, dans lequel chacune des préparations à usage local est contenue dans l'un des puits, chaque puits contient une concentration différente de niacine ou d'ester, et la bande flexible est adaptée pour application à la peau d'un patient et pour amener chacune des préparations à usage local en contact avec la peau.

13. Dispositif selon la revendication 12, dans lequel la bande flexible est hermétiquement fermée avec une pellicule de couverture détachable.

14. Dispositif pour diagnostiquer ou contrôler la schizophrénie comprenant : une préparation à usage local contenant de la niacine sous une forme efficace quelconque en une concentration dans la plage de 0,00001 M à 1 M procurant l'absorption de la niacine à travers la peau; et une aide de quantification pour noter la réaction de rougissement de la peau.

15. Dispositif pour diagnostiquer ou contrôler la schizophrénie comprenant : une préparation à usage local contenant un ester alkylique C₁-C₁₀ de niacine sous une forme efficace quelconque en une concentration dans la plage de 0,00001 M à 1 M procurant l'absorption de l'ester de niacine à travers la peau; et une aide de quantification pour noter la réaction de rougissement de la peau.

16. Dispositif selon la revendication 14 ou 15, dans lequel l'aide de quantification est un nuancier.
